# EUROPEAN PATENT SPECIFICATION

(11) **EP 2 619 341 B1**
(45) Date of publication and mention of the grant of the patent: **13.06.2018**
(21) Application number: 11760918.0
(22) Date of filing: 07.09.2011
(51) Int. Cl.: C22C 14/00, C22F 1/18, A61L 27/06, B62D 29/00

(54) **METHOD TO PRODUCE HIGH STRENGTH ALPHA/BETA TITANIUM ALLOY FASTENERS**
VERFAHREN ZUR HERSTELLUNG EINES BEFESTIGERMATERIAL AUS EINER HOCHFESTEN ALPHA-/BETA-TITANLEGIERUNG
MÉTHODE POUR PRODUIRE UNE PIÈCE DE FIXATION EN ALLIAGE DE TITANE ALPHA/BÊTA À HAUTE RÉSISTANCE

(30) Priority: 13.10.2010 US 903851; 23.09.2010 US 888699
(43) Date of publication of application: 31.07.2013
(73) Proprietor: ATI Properties LLC, Albany OR 97321 (US)
(72) Inventor: BRYAN, David J., Indian Trail North Carolina 28079 (US)
(74) Representative: Potter Clarkson LLP
(86) International application number: PCT/US2011/050595
(87) International publication number: WO 2012/039927

(56) References cited:
- WO-A1-2004/101838
- US-A- 5 980 655
- US-A1- 2003 211 003
- Anonymous: "ATI 425® Alloy Applications", ATI Webpage , 4 July 2010 (2010-07-04), XP002663645, Retrieved from the Internet: URL:http://www.alleghenytechnologies.com/a ti425/applications/default.asp [retrieved on 2011-11-15] -& ATI: "ATI 425® Alloy Applications", Printout of WayBack Machine , 4 July 2011 (2011-07-04), XP002663646, Retrieved from the Internet: URL:http://web.archive.org/web/20100704044 024/http://www.alleghenytechnologies.com/A TI425/applications/default.asp#other [retrieved on 2011-11-15]
- Anonymous: "Technical Data Sheet. ATI 425 Alloy (Version 1)", ATI webpage , 3 July 2010 (2010-07-03), pages 1-5, XP002663207, Retrieved from the Internet: URL:http://www.alleghenytechnologies.com/a ti425/specifications/datasheet.asp [retrieved on 2011-11-10] -& ATI: "Technical Data Sheet. ATI 425 Alloy (Version 1) - Printout of the screen", Internet Archive Wayback Machine , 3 July 2010 (2010-07-03), XP002663398, Retrieved from the Internet: URL:http://web.archive.org/web/20100703120 218/http://www.alleghenytechnologies.com/A TI425/specifications/datasheet.asp [retrieved on 2011-11-10] -& D.Greenfield: "ATI Aerospace presents results of year-long characterization program for the new ATI 425 Alloy Titanium products at Aeromat 2010", ATI Website: News release , 21 June 2010 (2010-06-21), XP002663402, Retrieved from the Internet: URL:http://www.alleghenytechnologies.com/a ti425/docs/Aeromat

## Description

### BACKGROUND OF THE TECHNOLOGY

### FIELD OF THE TECHNOLOGY

The present disclosure relates to mechanical fasteners and fastener stock, and in particular to fasteners and fastener stock comprising alpha/beta titanium alloys.

### DESCRIPTION OF THE BACKGROUND OF THE TECHNOLOGY

Titanium alloys typically exhibit a high strength-to-weight ratio, are corrosion resistant, and are resistant to creep at moderately high temperatures. For these reasons, titanium alloys are used in aerospace and aeronautic applications including, for example, landing gear members, engine frames, and mechanical fasteners.

Reducing the weight of an aircraft results in fuel savings, and thus there is a strong drive in the aerospace industry to reduce aircraft weight. Titanium and titanium alloys are attractive materials for achieving weight reduction in aircraft applications because of their high strength-to-weight ratio. Currently, titanium alloy
fasteners are used in less demanding aerospace applications. In certain aerospace applications in which titanium alloys do not exhibit sufficient strength to meet the particular mechanical requirements of the application, heavier iron and nickel based alloy fasteners are used.

Most titanium alloy parts used in aerospace applications are made from Ti-6AI-4V alloy (ASTM Grade 5; UNS R56400; AMS 4965), which is an alpha/beta titanium alloy. Typical minimum specification for small diameter Ti-6AI-4V fastener stock, i.e., fastener stock having a diameter less than 0.5 inches (1.27 cm), are 170 ksi (1,172 MPa) ultimate tensile strength (UTS), as determined according to ASTM E8/E8M-09 ("Standard Test Methods for Tension Testing of Metallic Materials" ASTM International, 2009), and 103 ksi (710 MPa) double shear strength (DSS), as determined according to NASM 1312-13 ("Method 13-Double Shear", Aerospace Industries Association-National Aerospace Standard (Metric), February 1, 2003).

Iron and nickel based superalloys, such as, for example, A286 iron-base superalloy (UNS S66286), are representative of materials used in aerospace fastener applications having the next tier of strength. Typical specified minimum strengths for cold drawn and aged A286 alloy fasteners are 180 ksi (1,241 MPa) UTS and 108 ksi (744 MPa) DSS.

Alloy 718 nickel based superalloy (N07718) is a material used in aerospace fasteners that represents the uppermost tier of strength. Typical specification minimums for cold drawn and aged Alloy 718 superalloy fasteners are 220 ksi (1,517 MPa) UTS and 120 ksi (827 MPa) DSS.

In addition, two beta titanium alloys that currently are in use or are under consideration for use as high strength fastener materials exhibit minimum ultimate tensile strength of 180 ksi (1,241.1 MPa) and minimum DSS of 108 ksi (744.6 MPa). SPS Technologies, Jenkintown, Pa., offers a titanium alloy fastener fabricated from an optimized beta-titanium alloy that conforms to the chemistry of Ti-3AI-8V-6Cr-4Zr-4Mo titanium alloy (AMS 4958). The SPS bolts are available in diameters up to 1 inch (2.54 cm). Alcoa Fastening Systems (AFS) has developed a high-strength
titanium fastener made from a titanium alloy that conforms to the nominal chemistry of Ti-5AI-5Mo-5V-3Cr-0.5Fe titanium alloy (also referred to as Ti-5553; UNS unassigned), a near beta-titanium alloy. The AFS Ti-5553 alloy fasteners reportedly exhibit tensile strength of 190 ksi (1,309 MPa), greater than 10% elongation, and minimum DSS of 113 ksi (779 MPa) for uncoated parts and 108 ksi (744 MPa) for coated parts.

Beta-titanium alloys generally include a high alloying content, which increases the cost of components and processing compared with alpha/beta titanium alloys. Beta-titanium alloys also generally have a higher density than alpha/beta titanium alloys. For example ATI 425® alpha/beta titanium alloy has a density of about 0.161 lbs/in³ (4.5 g/cm³), whereas the beta-titanium alloy Ti-3AI-8V-6Cr-4Zr-4Mo has a density of about 0.174 lbs/in³ (4.8 g/cm³), and the near beta-titanium alloy Ti-5AI-5Mo-5V-3Cr-0.5Fe has a density of about 0.168 lbs/in³ (4.7 g/cm³). Fasteners made from titanium alloys that are less dense may provide further weight savings for aerospace applications. In addition, the bimodal microstructure that is obtained, for example, in solution treated and aged alpha/beta titanium alloys may provide improved mechanical properties such as high cycle fatigue compared to beta-titanium alloys. Alpha/beta titanium alloys also have a higher beta transus temperature (Tp) than beta-titanium alloys. For example, the T_{β} of ATI 425® alpha/beta titanium alloy is about 1,800°F (982.2°C), whereas Ti-5Al-5Mo-5V-3Cr-0.5Fe beta titanium alloy has a T_{β} of about 1,500°F (815.6°C). The difference in T_{β} for the two forms of titanium alloys allows for a larger temperature window for thermomechanical processing and heat treatment in the alpha/beta phase field for alpha/beta titanium alloys.

Given the continuing need for reduced fuel consumption through aircraft weight reduction, a need exists for improved lightweight fasteners for aerospace applications. In particular, it would be advantageous to provide lightweight alpha/beta titanium alloy aerospace fasteners and fastener stock exhibiting higher strength than current generation aerospace fasteners fabricated from Ti-6AI-4V alpha/beta titanium alloy.

### SUMMARY

The invention provides a method for producing a titanium alloy fastener stock having a diameter of 4.57 mm (0.18 inches) to 31.8 mm (1.25 inches) in accordance with claim 1 of the appended claims.

The present disclosure describes an article of manufacture selected from a titanium alloy fastener and a titanium alloy fastener stock includes an alpha/beta titanium alloy comprising, in percent by weight: 3.9 to 4.5 aluminum; 2.2 to 3.0 vanadium; 1.2 to 1.8 iron; 0.24 to 0.3 oxygen; up to 0.08 carbon; up to 0.05 nitrogen; titanium; and up to a total of 0.3 of other elements. In a non-limiting embodiment, the alpha/beta titanium alloy fastener or fastener stock exhibits an ultimate tensile strength of at least 170 ksi (1,172 MPa) and a double shear strength of at least 103 ksi (710.2 MPa).

In an additional embodiment described, an article of manufacture selected from a titanium alloy fastener and a titanium alloy fastener stock comprises an alpha/beta titanium alloy consisting essentially of, in percent by weight: 3.9 to 4.5 aluminum; 2.2 to 3.0 vanadium; 1.2 to 1.8 iron; 0.24 to 0.3 oxygen; up to 0.08 carbon; up to 0.05 nitrogen; up to a total of 0.3 of other elements; titanium; incidental impurities; and wherein the other elements consist essentially of one or more of tin, zirconium, molybdenum, chromium, nickel, silicon, copper, niobium, tantalum, manganese, and cobalt, wherein the weight percent of each such element is 0.1 or less, and boron and yttrium, wherein the weight percent of each such element is less than 0.005. In a non-limiting embodiment, the alpha/beta titanium alloy fastener or fastener stock exhibits an ultimate tensile strength of at least 170 ksi (1,172 MPa) and a double shear strength of at least 103 ksi (710.2 MPa).

In an embodiment according to the present disclosure, a method for producing a titanium alloy fastener stock includes providing an alpha/beta titanium alloy comprising, in percent by weight: 3.9 to 4.5 aluminum; 2.2 to 3.0 vanadium; 1.2 to 1.8 iron; 0.24 to 0.3 oxygen; up to 0.08 carbon; up to 0.05 nitrogen; titanium; and up to a total of 0.3 of other elements. The alpha/beta titanium alloy is hot rolled and, subsequently, is annealed at an annealing temperature in a range of 1,200°F (648.9°C) to 1,400°F (760°C) for an annealing time in a range of 1 hour to 2 hours. After annealing, the alpha/beta titanium alloy is air cooled, and then machined to predetermined dimensions. The alpha/beta titanium alloy is then solution treated at a
solution treatment temperature in a range of 1,500°F (815.6°C) to 1,700°F (926.7°C) for a solution treating time in a range of 0.5 hours to 2 hours. After solution treatment, the alpha/beta titanium alloy is cooled at a cooling rate that is at least as fast as air cooling, and then aged at an aging treatment temperature in a range of 800°F (426.7°C) to 1,000°F (537.8°C) for an aging time in a range of 4 hours to 16 hours. Following aging, the titanium alloy is air cooled. In a non-limiting embodiment, an alpha/beta titanium alloy made according to the foregoing method embodiment exhibits an ultimate tensile strength of at least 170 ksi (1,172 MPa) and a double shear strength of at least 103 ksi (710.2 MPa).

### BRIEF DESCRIPTION OF THE DRAWINGS

The features and advantages of methods described herein may be better understood by reference to the accompanying drawings in which:
FIG. 1 is schematic representation of embodiments of fasteners described;
FIG. 2. is a flow diagram of an embodiment of a method of producing fasteners and fastener stock according to the present disclosure;
FIG. 3 is a plot of ultimate tensile strength of fastener bar and wire stock made by non-limiting embodiments according to the present disclosure, comparing those properties with requirements for Ti-6AI-4V titanium alloy fastener bar and wire stock;
FIG. 4 is a plot of yield strength of fastener bar and wire stock made by non-limiting embodiments according to the present disclosure, comparing those properties with requirements for Ti-6AI-4V titanium alloy fastener bar and wire stock; and
FIG. 5 is a plot of percent elongation of fastener bar and wire stock made by non-limiting embodiments according to the present disclosure, comparing those properties with requirements for Ti-6AI-4V titanium alloy fastener bar and wire stock.

The reader will appreciate the foregoing details, as well as others, upon considering the following detailed description of certain non-limiting embodiments of methods according to the present disclosure.

### DETAILED DESCRIPTION OF CERTAIN NON-LIMITING EMBODIMENTS

In the present description of non-limiting embodiments, other than in the operating examples or where otherwise indicated, all numbers expressing quantities or characteristics are to be understood as being modified in all instances by the term "about". Accordingly, unless indicated to the contrary, any numerical parameters set forth in the following description are approximations that may vary depending on the desired properties one seeks to obtain in the materials and by the methods according to the present disclosure. At the very least, and not as an attempt to limit the application of the doctrine of equivalents to the scope of the claims, each numerical parameter should at least be construed in light of the number of reported significant digits and by applying ordinary rounding techniques.

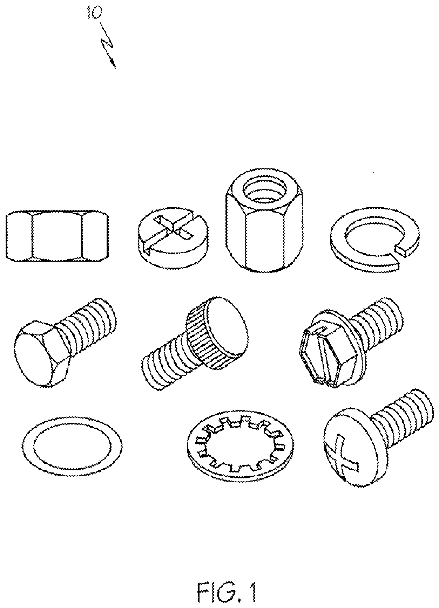

Referring now to FIG. 1, an aspect of this disclosure is directed to an article of manufacture selected from a titanium alloy fastener 10 and a titanium alloy fastener stock (not shown). In a non-limiting embodiment, the article includes an alpha/beta titanium alloy comprising, in percent by weight: 3.9 to 4.5 aluminum; 2.2 to 3.0 vanadium; 1.2 to 1.8 iron; 0.24 to 0.3 oxygen; up to 0.08 carbon; up to 0.05 nitrogen;
titanium; and up to a total of 0.3 of other elements. In non-limiting embodiments of this disclosure the other elements referred to in the alloy composition comprise or consist essentially of one or more of tin, zirconium, molybdenum, chromium, nickel, silicon, copper, niobium, tantalum, manganese, and cobalt, each having a maximum concentration of 0.1 weight percent as individual elements, and boron and yttrium, each having a maximum concentration of 0.005% as individual elements, with the sum total of all of the other elements not exceeding 0.3 weight percent. In a non-limiting embodiment, the alpha/beta titanium article of manufacture according to the present disclosure exhibits an ultimate tensile strength of at least 170 ksi (1,172 MPa) and a double shear strength (DSS) of at least 103 ksi (710.2 MPa) for fasteners having diameters in the range of 0.18 inches (4.57 mm) to 1.25 inches (31.8 mm). In a non-limiting embodiment of this disclosure, fasteners may have diameters as small as can be fabricated. In a non-limiting embodiment, fasteners according to the present disclosure exhibit a percent elongation of at least 10%.

In certain non-limiting embodiments, the elemental composition of an alpha/beta titanium alloy included in the fastener or fastener stock according to the present disclosure is encompassed by the alloy composition disclosed in U.S. Pat. No. 5,980,655 ("the '655 patent"). The '655 patent discloses an alloy having the composition shown in the following Table 1.

**Table 1**

| Alloying Element | Percent by Weight |
|---|---|
| Aluminum | from about 2.9 to about 5.0 |
| Vanadium | from about 2.0 to about 3.0 |
| Iron | from about 0.4 to about 2.0 |
| Oxygen | greater than 0.2 to about 0.3 |
| Carbon | from about 0.005 to about 0.03 |
| Nitrogen | from about 0.001 to about 0.02 |
| Other elements | less than about 0.5 |

A commercial version of the alloy of the '655 patent is ATI 425® alloy, which is available from ATI Aerospace, a business of Allegheny Technologies Incorporated, Pittsburgh, PA. The ultimate tensile strength of alloys having the elemental composition disclosed in the '655 patent ranges from about 130 to 133 ksi (896 to 917 MPa). However, the present inventor surprisingly discovered that the significantly narrower range of chemistry in the present disclosure results in alpha/beta titanium fasteners that may exhibit the significantly higher ultimate tensile strengths disclosed herein. In a non-limiting embodiment, the ultimate tensile strength of the fasteners disclosed herein, made from the alloy composition disclosed herein, was up to 22% greater than the UTS disclosed in the '655 patent. Without intending to be bound by any theory of operation, it is believed that the surprisingly high strength of fastener alloy compositions disclosed herein may have been at least in part a result of significantly increasing the aluminum and oxygen levels above minimum levels disclosed in the '655 patent, which may have increased the strength of the dominant alpha phase in the alpha/beta titanium alloy.

The inventor also surprisingly discovered that narrowing the allowable ranges of aluminum, vanadium, iron, oxygen, carbon, and nitrogen in the fastener alloy disclosed herein relative to the alloy disclosed in the '655 patent reduces the variability of the mechanical properties and the variability of the beta transus temperature of the fastener alloy disclosed herein. This reduced variability is important for process and microstructural optimization to achieve the superior mechanical properties disclosed herein.

In another non-limiting embodiment, a titanium alloy fastener and a titanium alloy fastener stock disclosed herein comprises a diameter of up to 0.75 inches (1.91 cm), and has an ultimate tensile strength of at least 180 ksi (1,241 MPa) and a double shear strength of at least 108 ksi (744.6 MPa). In a non-limiting embodiment, fasteners or fastener stock according to this disclosure have up to about 26% greater ultimate tensile strength than the ultimate tensile strength disclosed in the '655 patent.

Referring again to FIG. 1, according to another non-limiting aspect of this disclosure, an article of manufacture selected from a titanium alloy fastener 10 and a titanium alloy fastener stock (not shown) includes an alpha/beta titanium alloy consisting essentially of, in percent by weight: 3.9 to 4.5 aluminum; 2.2 to 3.0 vanadium; 1.2 to 1.8 iron; 0.24 to 0.3 oxygen; up to 0.08 carbon; up to 0.05 nitrogen; no more than a total of 0.3 of other elements; with the remainder titanium; and incidental impurities. In non-limiting embodiments of this disclosure the other elements referred to in the alloy composition comprise or consist essentially of one or more of tin, zirconium, molybdenum, chromium, nickel, silicon, copper, niobium, tantalum, manganese, and cobalt, wherein the weight percent of each such element is 0.1 or less, and boron and yttrium, wherein the weight percent of each such element is less than 0.005, with the sum total of all of the other elements not exceeding 0.3 weight percent. In a non-limiting embodiment, the article of manufacture has an ultimate tensile strength of at least 170 ksi (1,172 MPa) and a double shear strength of at least 103 ksi (710.2 MPa).

In a non-limiting embodiment, a titanium fastener and a titanium alloy fastener stock according to the present disclosure comprises a diameter of up to 0.75 inches (1.91 cm), an ultimate tensile strength of at least 180 ksi (1,241 MPa), and a double shear strength of at least 108 ksi (744.6 MPa).

As used herein, the term "fastener" refers to a hardware device that mechanically joins or affixes two or more objects together. A fastener includes, but is not limited to, a bolt, a nut, a stud, a screw, a rivet, a washer, and a lock washer. As used herein, the phrase "fastener stock" refers to an article that is processed to form one or more fasteners from the article.

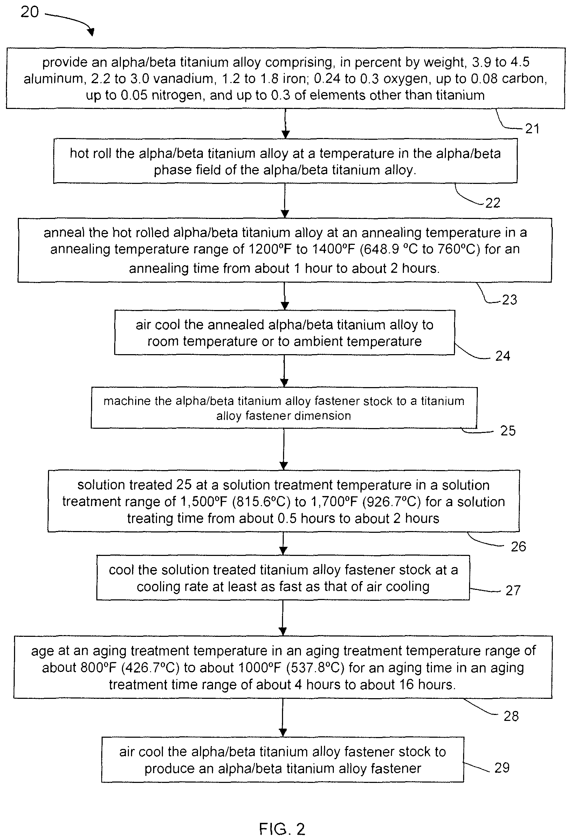

Referring to FIG. 2, a non-limiting aspect according of the present disclosure is a method 20 for producing a titanium alloy fastener or fastener stock. The method comprises providing 21 an alpha/beta titanium alloy comprising, in percent by weight: 3.9 to 4.5 aluminum; 2.2 to 3.0 vanadium; 1.2 to 1.8 iron; 0.24 to 0.3 oxygen; up to 0.08 carbon; up to 0.05 nitrogen; titanium; and up to a total of 0.3 of other elements. In non-limiting embodiments of this disclosure the other elements referred to in the alloy composition comprise or consist essentially of one or more of tin, zirconium, molybdenum, chromium, nickel, silicon, copper, niobium, tantalum, manganese, and cobalt, wherein the weight percent of each such element is 0.1 or less, and boron and yttrium, wherein the weight percent of each such element is less than 0.005, with the sum total of all of the other elements not exceeding 0.3 weight percent. The alpha/beta titanium alloy is hot rolled 22 at a temperature in the alpha/beta phase field of the alpha/beta titanium alloy. In a non-limiting embodiment, a hot rolling temperature is at least 50°F (27.8°C) below the beta transus temperature of the alpha/beta titanium alloy, but no more that 600°F (333.3°C) below the beta transus temperature of the alpha/beta titanium alloy.

After hot rolling 22, the alpha/beta titanium alloy optionally is cold drawn and annealed to reduce size without substantially changing the mechanical properties of the alpha/beta titanium alloy. In a non-limiting embodiment, cold drawing reduces the cross-sectional area of the titanium alloy workpiece by less than 10%. Prior to cold drawing, the alpha/beta titanium alloy may be coated with a solid lubricant, such as, but not limited to, molybdenum disulfide (MoS₂).

In a non-limiting embodiment, after hot rolling 22, the alpha/beta titanium alloy is annealed 23 and cooled 24 to provide an alpha/beta titanium alloy fastener stock. In a non-limiting embodiment, annealing 23 includes annealing the hot rolled alpha/beta titanium alloy at an annealing temperature in an annealing temperature range of 1,200°F to 1,400°F (649°C to 760°C). In another non-limiting embodiment, an annealing time ranges from about 1 hour to about 2 hours. In still another non-limiting embodiment, annealing 23 comprises annealing the hot rolled alpha/beta titanium alloy at about 1,275°F (690.6°C) for about one hour. In a non-limiting embodiment, after annealing 23, the annealed alpha/beta titanium alloy is cooled 24 to room temperature or to ambient temperature. In certain non-limiting embodiments, after annealing 23, the annealed alpha/beta titanium alloy is air cooled or water cooled to room temperature or to ambient temperature.

After annealing 23 and cooling 24, in a non-limiting embodiment, the alpha/beta titanium alloy fastener stock is machined 25 to a dimension useful for forming a fastener from the stock. Optionally, a coating may be applied to the alpha/beta titanium alloy fastener stock prior to machining. Conventional machining coatings are known to persons skilled in the art and need not be elaborated upon herein.

In a non-limiting embodiment, the machined titanium alloy fastener stock is solution treated 26 at a solution treatment temperature in a solution treatment range of 1,500°F (815.6°C) to 1,700°F (926.7°C) for a solution treating time in a range of 0.5 hours to 2 hours. In a specific non-limiting embodiment, the machined titanium alloy fastener stock is solution treated 26 at a solution treatment temperature of about 1610°F (876.7°C).

After solution treatment 26, the machined titanium alloy fastener stock is cooled 27. In non-limiting embodiments, cooling 27 may be carried out using, air cooling, water cooling, and/or water quenching, and may be referred to as "fast cooling". Preferably, the cooling rate achieved during cooling 27 is as fast as air cooling. In a non-limiting embodiment, cooling 27 comprises a cooling rate of at least 1,000°F (555.6°C) per minute. In a non-limiting embodiment, cooling 27 comprises any cooling process known to a person skilled in the art that achieves the indicated cooling rate. Fast cooling 27 is used to preserve the microstructure obtained by solution treatment 26.

In a non-limiting embodiment, the solution treated 26 and fast cooled 27 titanium alloy fastener stock is aged 28 at an aging treatment temperature in an aging treatment temperature range of about 800°F (426.7°C) to about 1,000°F (537.8°C) for an aging time in an aging treatment time range of about 4 hours to about 16 hours. In a specific non-limiting embodiment, the solution treated 26 and fast cooled 27 titanium alloy fastener stock is aged 28 at 850°F (454.4°C) for 10 hours. In certain non-limiting embodiments, after aging 28, the alpha/beta titanium alloy fastener stock is air cooled 29 or fast cooled to produce an alpha/beta titanium alloy fastener as disclosed herein.

It has been determined that fastener stock manufactured according to this disclosure has higher mechanical properties compared with fastener stock fabricated from Ti-6-4 titanium alloy. Therefore, it is possible to use fasteners fabricated according to this disclosure in smaller dimensions to replace Ti-6-4 fasteners in the same applications. This leads to savings in weight, which is of value in aerospace applications. It also has been determined that in certain applications, fasteners fabricated according to this disclosure could replace steel alloy fasteners having the same dimensions and result in a weight savings of value for aerospace applications.

The examples that follow are intended to further describe certain non-limiting embodiments, without restricting the scope of the present invention. Persons having ordinary skill in the art will appreciate that variations of the following examples are possible within the scope of the invention, which is defined solely by the claims.

### EXAMPLE 1

An ingot was produced from compacts made from raw materials using double vacuum arc remelt (VAR) technology. Samples were taken from the ingot for chemical analysis, and the measured average chemistry of the ingot is provided in Table 2. The beta transus temperature of the alloy was determined to be 1,785°F (973.9°C).

**Table 2**

| Al | V | Fe | O | N | C | Remainder |
|---|---|---|---|---|---|---|
| 4.06 | 2.52 | 1.71 | 0.284 | 0.008 | 0.017 | Ti and incidental impurities |

### EXAMPLE 2

Titanium alloy ingot from several heats having chemical compositions according to this disclosure were hot rolled at a hot rolling temperature of about 1,600°F (871.1°C). The hot rolled material was annealed at 1,275°F (690.6°C) for 1 hour and air cooled. The annealed material was machined into fastener stock bars and wires having various diameters from about 0.25 inches (6.35 mm) to about 3.5 inches (88.9 mm). The fastener stock bars and wires were solution treated at about 1,610°F (876.7°C) for about 1 hour and water quenched. After solution treatment and water quenching, the fastener stock bars and wires were aged at about 850°F (454.4°C) for about 10 hours and air cooled.

### EXAMPLE 3

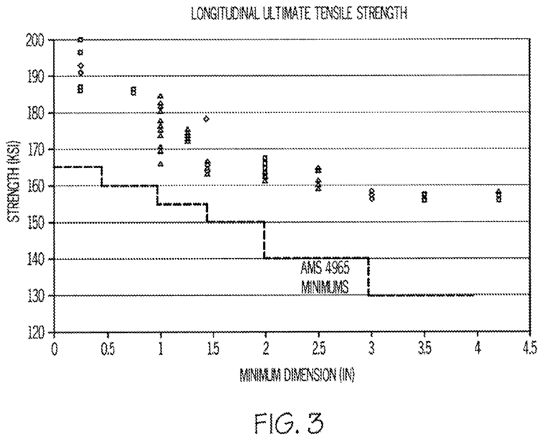

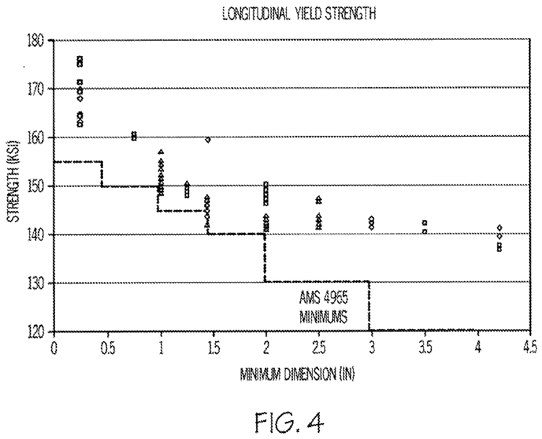

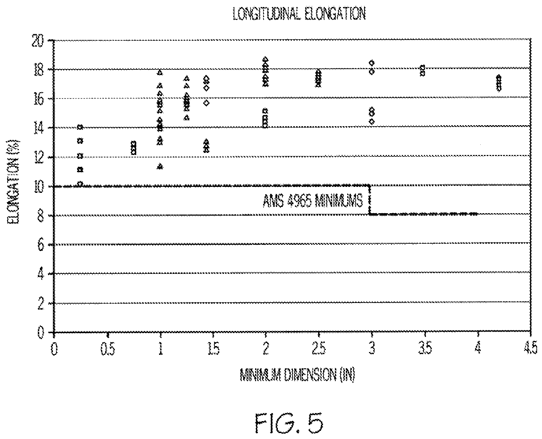

The fastener stock bars and wires from Example 2 were tensile tested at room temperature. The ultimate tensile strengths of the fastener stock bars and wires are presented graphically in FIG. 3. The yield strengths of the fastener stock bars and wires are presented graphically in FIG. 4, and the percent elongations of fastener stock bars and wires are presented graphically in FIG. 5. The minimum ultimate tensile strength, yield strength, and percent elongation required for solution treated and aged Ti-6AI-4V alloy in aerospace fastener applications (AMS 4965) are also illustrated in FIGS. 3-5, respectively. It is seen from FIG. 3 that ultimate tensile strengths measured for the fastener stock bar and wire manufactured according to this disclosure exceeded the illustrated Ti-6AI-4V alloy specifications by the significant amount of approximately 20 ksi (138 MPa) in all measured diameter sizes. Further, it is seen from FIG. 5 that fastener stock having chemical compositions according to this disclosure exhibited percent elongations in the range of at least 10 percent to about 19 percent.

### EXAMPLE 4

Fastener stock having a diameter of about 0.25 inches (6.35 mm), having the chemical composition from Example 1, and solution treated and aged as in Example 2 was tensile tested. The results of the tensile tests are listed in Table 3.

**Table 3**

| Ultimate Tensile Strength (ksi) | Yield Strength (ksi) | Percent Elongation | Reduction in Area | Double Shear Strength (ksi) |
|---|---|---|---|---|
| 199.9 | 175.1 | 13.0 | 45 | 123.3 |
| 199.9 | 176.2 | 13.0 | 44 | 120.0 |
| 196.3 | 169.4 | 10.0 | 39 | 117.4 |
| 196.9 | 171.4 | 11.0 | 39 | 117.2 |

The ultimate tensile strengths ranged from about 196 ksi to about 200 ksi (1351 MPa to 1379 MPa), which is higher than the minimum requirements for Ti-6AI-4V fastener stock of 170 ksi (1,172 MPa) UTS and 103 ksi (710 MPa) DSS. It is also observed that the properties agree with the accepted empirical relationship that DSS = 0.6 X UTS.

### EXAMPLE 5

Fastener stock having a diameter of about 0.75 inches (1.91 cm), having a chemistry from Example 1, and heat treated according to Example 2 was tensile tested. The results of the tensile tests are listed in Table 4.

**Table 4**

| Diameter (inch) | Ultimate Tensile Strength (ksi) | Yield Strength (ksi) | Percent Elongation |
|---|---|---|---|
| 0.75 | 185.9 | 160.3 | 12.3 |
| 0.75 | 185.8 | 160.1 | 12.8 |
| 0.75 | 185.4 | 159.7 | 12.9 |
| 0.75 | 186 | 159.5 | 12.7 |
| 0.75 | 186.1 | 160.3 | 12.4 |
| 0.75 | 186.1 | 160 | 12.4 |
| 0.75 | 186.3 | 160.6 | 12.4 |
| 0.75 | 186.1 | 160.3 | 12.8 |
| **Average** | **186.0** | **160.1** | **12.6** |
| **STD** | **0.3** | **0.4** | **0.2** |

The average ultimate tensile strength of the 0.75 inch (1.91 cm) fastener stock bars was 186 ksi (1,282 MPa), which satisfies the minimum specification for fasteners fabricated from A286 iron-base superalloy. Based upon the accepted empirical relationship between DSS and UTS presented hereinabove, the 0.75 inch (1.91 cm) bars are expected to also meet the 108 ksi (744 MPa) DSS requirement for fasteners fabricated from A286 iron-base superalloy.

### EXAMPLE 6

Ingot having the chemical composition as in Example 1 is hot rolled, annealed, and machined as in Example 2 to form a fastener stock having a diameter of about 0.75 inches (1.91 cm). The fastener stock is computer numerical control machined into a fastener having a shape of a stud. The stud is solution treated and aged as in Example 2 to form a non-limiting embodiment of a fastener of this disclosure.

### EXAMPLE 7

Ingot having the chemical composition as in Example 1 is hot rolled, annealed, and machined as in Example 2 to form a fastener stock having a diameter of about 1 inch (2.54 cm). The fastener stock is roll threaded and cut into pieces having lengths of about 2 inches (5.08 cm). The pieces are cold forged to form hex head bolts. The hex head bolts are solution treated and aged as in Example 2 to form a non-limiting embodiment of a fastener according to this disclosure.

### EXAMPLE 7

Ingot having the chemical composition as in Example 1 is hot rolled, annealed, and machined as in Example 2 to form a fastener stock having a diameter of about 1 inch (2.54 cm). The center of the fastener stock is machined to provide a 0.5 inch (1.27 cm) diameter hole. The fastener stock is then cut into pieces having a thickness of 0.125 inches (0.318 cm). The fastener stock is solution treated and aged as in Example 2 to form a non-limiting embodiment of a fastener in the form of a washer according to this disclosure.

The present disclosure has been written with reference to various exemplary, illustrative, and non-limiting embodiments. However, it will be recognized by persons having ordinary skill in the art that various substitutions, modifications, or combinations of any of the disclosed embodiments (or portions thereof) may be made without departing from the scope of the invention as defined solely by the claims. Thus, it is contemplated and understood that the present disclosure embraces additional embodiments not expressly set forth herein. Such embodiments may be obtained, for example, by combining and/or modifying any of the disclosed steps, ingredients, constituents, components, elements, features, aspects, and the like, of the embodiments described herein. Thus, this disclosure is not limited by the description of the various exemplary, illustrative, and non-limiting embodiments, but rather solely by the claims. In this manner, it will be understood that the claims may be amended during prosecution of the present patent application to add features to the claimed invention as variously described herein.

## Claims

1. A method for producing a titanium alloy fastener stock having a diameter from 4.57 mm (0.18 inches) to 31.8 mm (1.25 inches), the method comprising:
providing an alpha/beta titanium alloy comprising, in percent by weight:
3.9 to 4.5 aluminum;
2.2 to 3.0 vanadium;
1.2 to 1.8 iron;
0.24 to 0.3 oxygen;
up to 0.08 carbon;
up to 0.05 nitrogen;
up to a total of 0.3 of other elements, wherein the up to a total of 0.3% of other elements includes one or more of:
less than 0.005 each of boron and yttrium;
no greater than 0.1 each of tin, zirconium, molybdenum, chromium, nickel, silicon, copper, niobium, tantalum, manganese, and cobalt; and
balance titanium and incidental impurities;
hot rolling the titanium alloy in an alpha/beta phase of the titanium alloy; annealing the titanium alloy at an annealing temperature in a range of 648.9ºC (1200ºF) to 760ºC (1400ºF) for an annealing time in a range of 1 hour to 2 hours;
air cooling the titanium alloy;
machining the titanium alloy to a predetermined dimension of a diameter from 4.57 mm (0.18 inches) to 31.8 mm (1.25 inches); solution treating the machined titanium alloy in a solution treatment range of 815.6ºC (1500ºF) to 926.7ºC (1700ºF) for a solution treating time in a range of 0.5 hours to 2 hours;
cooling the titanium alloy at a cooling rate of at least 555.6°C (1000°F) per minute;
aging the titanium alloy at an aging treatment temperature in a range of 426.7ºC (800ºF) to 537.8ºC (1000ºF) for a solution treating time in a range of 4 hours to 16 hours; and
air cooling the titanium alloy.

2. The method of claim 1, wherein the titanium alloy is machined to a dimension of a diameter up to 19.1 mm (0.75 inches).

3. The method of claim 1 or claim 2, wherein the hot rolling is conducted at a temperature in the range of 27.8ºC (50ºF) below a beta transus temperature of the titanium alloy to 333.3ºC (600ºF) below the beta transus temperature of the titanium alloy.

4. The method of claim 1 or claim 2, further comprising after hot rolling and before annealing the titanium alloy, cold drawing the titanium alloy to a reduction in cross-section area less than 10% and annealing.

5. The method of claim 4, further comprising coating the titanium alloy with a solid lubricant before drawing.

6. The method of claim 5, wherein the solid lubricant is molybdenum disulfide.

7. The method of claim 1 or claim 2, wherein the annealing temperature is 690.6ºC (1275ºF) and the annealing time is 1 hour.

8. The method of claim 1 or claim 2, wherein the titanium alloy is coated prior to machining the titanium alloy.

9. The method of claim 1 or claim 2, wherein cooling after the solution treating step comprises one of air cooling, water cooling, and water quenching.

10. The method of claim 1 or claim 2, wherein the solution treatment temperature is 876.7ºC (1610ºF) and cooling the titanium alloy comprises water quenching.

11. The method of claim 1 or claim 2, wherein the aging the titanium alloy comprises aging at 454.4ºC (850ºF) for 10 hours.

## Patentansprüche

1. Verfahren zum Herstellen eines Befestigungselement-Materials aus Titanlegierung, das einen Durchmesser von 4,57 mm (0,18 Zoll) bis 31,8 mm (1,25 Zoll) hat, wobei das Verfahren Folgendes umfasst:
Bereitstellen einer Alpha/Beta-Titanlegierung, die, in Gewichtsprozent, Folgendes umfasst:
3,9 bis 4,5 Aluminium,
2,2 bis 3,0 Vanadium,
1,2 bis 1,8 Eisen,
0,24 bis 0,3 Sauerstoff,
bis zu 0,08 Kohlenstoff,
bis zu 0,05 Stickstoff,
bis zu insgesamt 0,3 an anderen Elementen,
wobei die bis zu insgesamt 0,3 % an anderen Elementen eines oder mehrere von Folgendem einschließen:
weniger als 0,005 jeweils von Bor und Yttrium,
nicht mehr als 0,1 jeweils von Zinn, Zirkonium, Molybdän, Chrom, Nickel, Silizium, Kupfer, Niob, Tantal, Mangan und Kobalt und
Rest Titan und Nebenverunreinigungen,
Warmwalzen der Titanlegierung in einer Alpha/Beta-Phase der Titanlegierung,
Glühen der Titanlegierung bei einer Glühtemperatur in einem Bereich von 648,9°C (1200°F) bis 760°C (1400°F) für eine Glühzeit in einem Bereich von 1 Stunde bis 2 Stunden,
Luftkühlen der Titanlegierung,
Spanen der Titanlegierung auf eine vorbestimmte Abmessung eines Durchmessers von 4,57 mm (0,18 Zoll) bis 31,8 mm (1,25 Zoll),
Lösungsbehandeln der gespanten Titanlegierung in einem Lösungsbehandlungsbereich von 815,6°C (1500°F) bis 926,7°C (1700°F) für eine Lösungsbehandlungszeit in einem Bereich von 0,5 Stunden bis 2 Stunden,
Kühlen der Titanlegierung mit einer Kühlungsgeschwindigkeit von wenigstens 555,6°C (1000°F) pro Minute,
Aushärtenlassen der Titanlegierung bei einer Aushärtungsbehandlungstemperatur in einem Bereich von 426,7°C (800°F) bis 537,8°C (1000°F) für eine Lösungsbehandlungszeit in einem Bereich von 4 Stunden bis 16 Stunden und
Luftkühlen der Titanlegierung.

2. Verfahren nach Anspruch 1, wobei die Titanlegierung auf eine Abmessung eines Durchmessers von bis zu 19,1 mm (0,75 Zoll) gespant wird.

3. Verfahren nach Anspruch 1 oder Anspruch 2, wobei das Warmwalzen bei einer Temperatur in dem Bereich von 27,8°C (50°F) unterhalb einer Beta-Transustemperatur der Titanlegierung bis 333,3°C (600°F) unterhalb der Beta-Transustemperatur der Titanlegierung durchgeführt wird.

4. Verfahren nach Anspruch 1 oder Anspruch 2, das ferner nach dem Warmwalzen und vor dem Glühen der Titanlegierung Kaltziehen der Titanlegierung auf eine Verringerung in der Querschnittsfläche von weniger als 10 % und Glühen umfasst.

5. Verfahren nach Anspruch 4, das ferner Beschichten der Titanlegierung mit einem festen Schmierstoff vor dem Ziehen umfasst.

6. Verfahren nach Anspruch 5, wobei der feste Schmierstoff Molybdändisulfid ist.

7. Verfahren nach Anspruch 1 oder Anspruch 2, wobei die Glühtemperatur 690,6°C (1275°F) beträgt und die Glühzeit 1 Stunde beträgt.

8. Verfahren nach Anspruch 1 oder Anspruch 2, wobei die Titanlegierung vor dem Spanen der Titanlegierung beschichtet wird.

9. Verfahren nach Anspruch 1 oder Anspruch 2, wobei das Kühlen nach dem Lösungsbehandlungsschritt eines von Luftkühlen, Wasserkühlen und Wasserabschrecken umfasst.

10. Verfahren nach Anspruch 1 oder Anspruch 2, wobei die Lösungsbehandlungstemperatur 876,7°C (1610°F) beträgt und das Kühlen der Titanlegierung Wasserabschrecken umfasst.

11. Verfahren nach Anspruch 1 oder Anspruch 2, wobei das Aushärtenlassen der Titanlegierung Aushärtenlassen bei 454,4°C (850°F) für 10 Stunden umfasst.

## Revendications

1. Procédé de production d'un matériau d'élément de fixation en alliage de titane ayant un diamètre de 4,57 mm (0,18 pouce) à 31,8 mm (1,25 pouce), le procédé comprenant :
la fourniture d'un alliage de titane alpha/bêta comprenant, en pourcentage en poids :
de 3,9 à 4,5 d'aluminium ;
de 2,2 à 3,0 de vanadium ;
de 1,2 à 1,8 de fer ;
de 0,24 à 0,3 d'oxygène ;
jusqu'à 0,08 de carbone ;
jusqu'à 0,05 d'azote ;
jusqu'à un total de 0,3 d'autres éléments, dans lequel le total de 0,3 % d'autres éléments comporte un ou plusieurs parmi :
moins de 0,005 chacun de bore et d'yttrium ;
pas plus de 0,1 chacun d'étain, de zirconium, de molybdène, de chrome, de nickel, de silicium, de cuivre, de niobium, de tantale, de manganèse et de cobalt ; et
le reste étant du titane et des impuretés accidentelles ;
le laminage à chaud de l'alliage de titane dans une phase alpha/bêta de l'alliage de titane ;
le recuit de l'alliage de titane à une température de recuit dans une plage de 648,9 °C (1 200 °F) à 760 °C (1 400 °F) pendant une durée de recuit dans une plage de 1 heure à 2 heures ;
le refroidissement à l'air de l'alliage de titane ;
l'usinage de l'alliage de titane jusqu'à une dimension prédéterminée d'un diamètre de 4,57 mm (0,18 pouce) à 31,8 mm (1,25 pouce) ;
le traitement de mise en solution de l'alliage de titane usiné dans une plage de traitement de mise en solution de 815,6 °C (1 500 °F) à 926,7 °C (1 700 °F) pendant une durée de traitement de mise en solution dans une plage de 0,5 heure à 2 heures ;
le refroidissement de l'alliage de titane à une vitesse de refroidissement d'au moins 555,6 °C (1 000 °F) par minute ;
le vieillissement de l'alliage de titane à une température de traitement de vieillissement dans une plage de 426,7 °C (800 °F) à 537,8 °C (1 000 °F) pendant une durée de traitement de mise en solution dans une plage de 4 heures à 16 heures ; et
le refroidissement à l'air de l'alliage de titane.

2. Procédé selon la revendication 1, dans lequel l'alliage de titane est usiné jusqu'à une dimension d'un diamètre jusqu'à 19,1 mm (0,75 pouce).

3. Procédé selon la revendication 1 ou la revendication 2, dans lequel le laminage à chaud est conduit à une température dans la plage de 27,8 °C (50 °F) en dessous d'une température transus bêta de l'alliage de titane à 333,3 °C (600 °F) en dessous de la température transus bêta de l'alliage de titane.

4. Procédé selon la revendication 1 ou la revendication 2, comprenant en outre après le laminage à chaud et avant le recuit de l'alliage de titane, l'étirage à froid de l'alliage de titane jusqu'à une réduction d'aire en coupe inférieure à 10 % et le recuit.

5. Procédé selon la revendication 4, comprenant en outre le revêtement de l'alliage de titane avec un lubrifiant solide avant l'étirage.

6. Procédé selon la revendication 5, dans lequel le lubrifiant solide est le disulfure de molybdène.

7. Procédé selon la revendication 1 ou la revendication 2, dans lequel la température de recuit est de 690,6 °C (1 275 °F) et la durée de recuit est 1 heure.

8. Procédé selon la revendication 1 ou la revendication 2, dans lequel l'alliage de titane est revêtu avant d'usiner l'alliage de titane.

9. Procédé selon la revendication 1 ou la revendication 2, dans lequel le refroidissement après l'étape de traitement de mise en solution comprend l'un parmi le refroidissement à l'air, le refroidissement à l'eau et la trempe à l'eau.

10. Procédé selon la revendication 1 ou la revendication 2, dans lequel la température de traitement de mise en solution est de 876,7 °C (1 610 °F) et le refroidissement de l'alliage de titane comprend la trempe à l'eau.

11. Procédé selon la revendication 1 ou la revendication 2, dans lequel le vieillissement de l'alliage de titane comprend le vieillissement à 454,4 °C (850 °F) pendant 10 heures.
